Europäisches Patentamt

European Patent Office

Office européen des brevets

(19)

(11) Veröffentlichungsnummer: **0 222 289 B1**

(12) # EUROPÄISCHE PATENTSCHRIFT

(45) Veröffentlichungstag der Patentschrift: **17.06.92**

(51) Int. Cl.⁵: **C07K 17/08**, C12N 11/08, C12N 11/04, C07K 17/04

(21) Anmeldenummer: **86115197.5**

(22) Anmeldetag: **03.11.86**

(54) **Verfahren zur Immobilisierung von biologischem Material, teilchenförmige Koagulate enthaltend ein immobilisiertes biologisches Material und ihre Verwendung als Katalysator.**

(30) Priorität: **14.11.85 DE 3540333**

(43) Veröffentlichungstag der Anmeldung:
**20.05.87 Patentblatt 87/21**

(45) Bekanntmachung des Hinweises auf die
Patenterteilung:
**17.06.92 Patentblatt 92/25**

(84) Benannte Vertragsstaaten:
**CH DE FR GB IT LI NL**

(56) Entgegenhaltungen:
**EP-A- 0 151 937
EP-A- 0 160 260
EP-A- 0 194 578
DE-A- 2 929 872
US-A- 4 312 946**

**PATENT ABSTRACTS OF JAPAN, Band 10,
Nr. 103 (C-340)[2160], 18. April 1986; & JP-
A-60 232 089 (TOYO GOMU KOGYO K.K.)
18-11-1985**

(73) Patentinhaber: **BAYER AG**

**W-5090 Leverkusen 1 Bayerwerk(DE)**

(72) Erfinder: **Lorenz, Otto, Prof. Dr.
Kurbrunnenstrasse 22
W-5100 Aachen(DE)**
Erfinder: **Reiff, Helmut, Dr.
Paul-Klee-Strasse 68i
W-5090 Leverkusen 1(DE)**
Erfinder: **Dieterich, Dieter, Dr.
H.T. -von-Böttinger-Strasse 16
W-5090 Leverkusen 1(DE)**

**Beschreibung**

Die Erfindung betrifft ein Verfahren zur Immobilisierung von biologischem Material in einer Polyurethanionomer-Matrix, das nach diesem Verfahren erhältliche immobilisierte biologische Material, sowie dessen Verwendung bei Biotransformationen.

Die Immobilisierung von Biokatalysatoren führt in der Regel zu verfahrenstechnischen und wirtschaftlichen Vorteilen. Es lassen sich chemische und physikalische Eigenschaften, manchmal auch Selektivität und Spezifität des Biokatalysators durch die Immobilisierung modifizieren.

Die Methoden zur Immobilisierung lassen sich allgemein in folgende Gruppen einteilen

- Adsorption an vorgefertigte Träger
- Kovalente Bindung an Träger
- Quervernetzung
- Einschluß in eine Polymermatrix.

Das Einschlußverfahren beruht darauf, daß das Biomaterial hinsichtlich seines Bewegungsraumes in ein Netzwerk mehr oder minder einheitlicher Maschen- und Porenweite eingeschlossen wird. Dieses Polymernetzwerk sollte für den Biokatalysator unpassierbar sein, während es dem Substrat und dem Produkt einer an diesem Katalysator durchgeführten Umsetzung praktisch keinen Widerstand bieten sollte.

Die Einbettung in eine polymere, hydrophile Matrix ist eine universell anwendbare Methode, da sie keine speziellen Wechselwirkungen zwischen Matrix und Biokatalysator voraussetzt. Erforderlich ist jedoch, daß die Matrix als Hydrogel eine hinreichende mechanische und chemische Stabilität gegenüber der vorgegebenen und der sich ausbildenden Umgebung besitzt. Für die Immobilisierung gewinnen zunehmend synthetische Polymere an Bedeutung. Dabei wird im allgemeinen von Monomeren oder Prepolymeren ausgegangen, die in Gegenwart des Biokatalysators in ein hochmolekulares lineares oder vernetztes Endprodukt überführt werden. Häufig wirken Monomere und auch Prepolymere toxisch auf den Biokatalysator, auch kann durch die bei der Polymerisation oder Polyaddition auftretende Wärmeentwicklung eine Desaktivierung des Biokatalysators erfolgen.

Herkömmliche Verfahren verwenden niedermolekulare, hydrophile Acrylat-Monomere zum Aufbau des Netzwerkes, beispielsweise Hydroxyethylacrylat, Hydroxyethylmethacrylat, Hydroxypropylacrylat, Hydroxypropylmethacrylat, Acrylamid u.a., die mit der wäßrigen Lösung oder Dispersion des Biomaterials gemischt und anschließend polymerisiert werden.

Verfahren, bei denen Polyurethan (PUR)-Vorprodukte eingesetzt wurden, gehen häufig von NCO-terminierten Prepolymeren aus. So verwendeten Fukui und Mitarbeiter (Adv. Biochem. Eng., Biotechnology 29 (1984) 1) wasserlösliche bzw. in Wasser emulgierbare Prepolymere aus Isophorondiisocyanat und Block-Copolyethern aus Ethylenoxid und Propylenoxid, die durch Reaktion mit Wasser unter Kettenverlängerung Hydrogele bilden; dabei ist die Hydrophilie des Endproduktes vom Ethylenoxidgehalt abhängig. Von den gleichen Autoren wurden NCO-terminierte Prepolymere mit 2-Hydroxyethylacrylat zu einem Vinyl-terminierten Vorprodukt umgesetzt, das unter Lichteinwirkung ein Netzwerk bildet. Das Umsetzungsprodukt aus überschüssigem Diisocyanat und 2-Hydroxyethylmethacrylat wurde zur Immobilisierung von Enzymen verwendet, dabei wurden die Reaktionsbedingungen so gewählt, daß covalente Bindungen zwischen NCO-Prepolymer und Enzym entstanden (M. Kumakura, I. Kaetsu, J. Disp. Sci. Technol. 4 (1983) 147).

EP-A-194578 (ein Dokument gemäß Art 54(3)) beschreibt Koagulate bestehend aus einem koagulierten Polyurethan-Kationomer.

Bei allen diesen Verfahren treten jedoch Schwierigkeiten bei der Steuerung und Reproduzierbarkeit der Permeabilitäten für das biolgische Material sowie für dessen Substrate und Produkte auf. Für eine Eignung als Immobilisierungsmaterial sind diese Größen jedoch essentiell. Außerdem ist die Verwendung niedermolekularer Acrylsäurederivate und/oder monomerer Diisocyanate wegen ihrer Toxität nachteilig. Es kann während der Polymerisation bzw. Polyaddition zu einer Schädigung des Biokatalysators kommen, oder im Polymerisat befindliche Restmonomere schränkten die Verwendung des immobilisierten Biokatalysators zur Herstellung von Produkten für die Nahrungsmittelindustrie oder für die Pharmazeutische Industrie stark ein.

Es wurde nun ein Verfahren zur Immobilisierung von biologischem Material durch Einschluß in eine Polymermatrix gefunden, das im wesentlichen darin besteht, daß man das gelöste oder emulgierte biologische Material mit der wäßrigen Lösung oder Dispersion eines Polyurethanionomers innig vermischt, die Mischung zu teilchenförmigen, vorzugsweise kugeligen Gebilden koaguliert und den so erhaltenen Biokatalysator wäscht und isoliert.

Gegenstand der Erfindung ist somit ein Verfahren zur Immobilisierung von biologischem Material in einer Polyurethan-Matrix, das dadurch gekennzeichnet ist, daß man eine wäßrige Lösung oder Dispersion des biologischen Materials mit einer wäßrigen Polyurethanionomer-Dispersion oder -Lösung mischt und anschließend die Mischung koaguliert.

Gegenstand der Erfindung sind auch teilchenförmige nach dem obigen Verfahren erhältliche Koagulate, bestehend aus einem koagulierten Polyurethanionomer und darin dispergiertem und immobilisiertem biologischem Material.

Gegenstand der Erfindung ist weiterhin die Verwendung von teilchenförmigen Koagulaten, bestehend aus einem koagulierten Polyurethanionomer und darin dispergiertem und immobilisiertem, biologischem Material zur Durchführung von Biotransformationen.

Durch die Verwendung des erfindungsgemäß immobilisierten biologischen Materials für Biotransformationen ergeben sich gegenüber dem Stand der Technik eine Fülle von Vorteilen, von denen einige genannt seien:

1. Polyurethanionomere lassen sich "maßschneidern" für spezielle Zwecke; so kann beispielsweise die gewünschte Hydrophilie eingestellt werden durch Art und Menge der ionischen Gruppen sowie der gegebenenfalls außerdem vorliegenden nichtionisch hydrophilen Segmente.

2. Ein weiterer Vorteil der erfindungsgemäßen Produkte ist die gute Reproduzierbarkeit und Steuerung der Permeabilitäten, die sich im wesentlichen aus der Hydrophilie-Hydrophobie-Bilanz der Polyurethan-Ionomeren ergeben. Die Verfahrensprodukte zeigen eine ausgezeichnete mechanische Stabilität.

3. Das biologische Material wird bei Raumtemperatur und in Wasser immobilisiert, gegebenenfalls bei physiologischen pH-Werten. Dies ist extrem schonend bezüglich beispielsweise der Aktivität von Enzymen.

4. Die Immobilisierung gelingt auch sicher bei Temperaturen unterhalb Raumtemperatur, beispielsweise bei +5°C bis +10°C; für hochempfindliche biologische Materialien kann dies eine sehr wichtige Voraussetzung sein.

5. Im Gegensatz zum Stand der Technik wird keine chemische Reaktion durchgeführt; das Verfahren arbeitet völlig monomerenfrei, da ein ausreagiertes PUR-Ionomer in Wasser verwendet wird. Insbesondere sind zu keinem Zeitpunkt monomere Isocyanate, Polyisocyanate oder NCO-Semiprepolymere anwesen, die gegebenenfalls auf empfindliche Enzyme toxisch wirken.

Das Problem der Desaktivierung durch toxische Verbindungen entfällt bei dem erfindungsgemäßen Verfahren also völlig.

6. Vorteilhaft ist auch die Möglichkeit, nach dem erfindungsgemäßen Verfahren schnell, sicher und wirtschaftlich große Mengen der immobilisierten biologischen Materialien herzustellen.

7. Die erfindungsgemäßen Verfahrensprodukte sind frei von gängigen Emulgatoren und Netzmitteln, so daß jegliche Migrationsprobleme aufgrund derartiger Zusatzmittel entfallen.

Für das erfindungsgemäße Verfahren können beliebige, stabile, wäßrige Lösungen oder vorzugsweise Dispersionen von ionisch modifizierten Polyurethanen eingesetzt werden, die nach den üblichen Methoden koaguliert werden können. Grundsätzlich ist es auch möglich, Mischungen verschiedener Polyurethandispersionen zur Herstellung der Gemische mit den gelösten oder dispergierten biologischen Materialien zu verwenden, sofern es sich um Dispersionen von gleichgeladenen Polyurethanen handelt. Die beim erfindungsgemäßen Verfahren einzusetzenden Polyurethandispersionen oder auch -lösungen weisen im allgemeinen einen Feststoffgehalt von 10 bis 60 Gew.-%, vorzugsweise von 10 bis 50 Gew.-%, und bei Raumtemperatur eine Viskosität von mindestens 50, vorzugsweise mindestens 300 mPa.s. auf. Der mittlere Teilchendurchmesser der dispergierten oder gelösten Polyurethane liegt im allgemeinen unter 1 $\mu$m, vorzugsweise bei 0,001 bis 0,5 und besonders bevorzugt bei 0,01 bis 0,3 $\mu$m. Die dispergierten oder gelösten Polyurethane weisen im übrigen einen die Dispergierbarkeit oder auch Löslichkeit in Wasser gewährleistenden Gehalt an ionischen Gruppen auf. Im Falle der kationisch modifizierten Polyurethane liegen in diesen im allgemeinen chemisch eingebaute quartäre Ammoniumgruppen und im Falle der anionisch modifizierten Polyurethane chemisch eingebaute Carboxylat- und/oder Sulfatgruppen vor. Der Gehalt an diesen ionischen Zentren liegt vorzugsweise bei 15 bis 300, besonders bevorzugt 30 bis 200 Milliäquivalenten pro 100 g Feststoff. Es ist auch möglich, wäßrige Dispersionen von solchen Polyurethanen einzusetzen, die neben diesen ionischen Zentren end- und/oder seitenständig angeordnete, Ethylenoxideinheiten aufweisende Polyetherketten als zusätzliche "interne Emulgatoren" enthalten, sofern hierdurch die erfindungswesentliche Koagulierbarkeit der Dispersionen nicht beeinträchtigt wird. Vorzugsweise werden jedoch beim erfindungsgemäßen Verfahren wäßrige Dispersionen von solchen Polyurethanen eingesetzt, deren Hydrophilie und damit Dispergierbarkeit ausschließlich oder überwiegend auf die Anwesenheit der genannten, eingebauten ionischen Zentren zurückzuführen ist.

Die Herstellung der beim erfindungsgemäßen Verfahren einzusetzenden Polyurethandispersionen oder auch Lösungen aus den üblichen Ausgangsmaterialien, d.h. aus organischen Polyisocyanaten, höhermolekularen Verbindungen mit gegenüber Isocyanatgruppen reaktionsfähigen Wasserstoffatomen und gegebenenfalls niedermolekularen Kettenverlängerungsmitteln unter Mitverwendung von, ionische Gruppen oder in ionische Gruppen überführbaren Gruppen aufweisenden Aufbaukomponenten und gegebenenfalls unter Mitverwendung von hydrophile Polyetherketten aufweisenden Aufbaukomponenten gehört zum bekannten Stand der Technik und ist beispielsweise in DE-PS 1 184 646, DE-PS 1 178 586, DE-AS 1 237 306, DE-OS 1 495 745, DE-OS 1 495 602, DE-OS 1 770 068, DE-OS 2 019 324, DE-OS 1 495 847, DE-OS 2 725 589, US-PS 4 269 748, US-PS 4 192 937, US-PS 4 292 226, US-PS 3 756 992 oder auch von D. Dieterich et al. in Angew. Chem., 82, 53-63 (1970) oder in Progress in Org. Coatings 9 281-300 (1981) beschrieben.

Es ist auch möglich, beim erfindungsgemäßen Verfahren Abmischungen von wäßrigen Dispersionen ionisch modifizierter Polyurethane mit gleichgeladenen Polymerisatdispersionen wie z.B. Polyvinylacetat-, Polyethylen-, Polystyrol-, Polybutadien-, Polyvinylchlorid-, Polyacrylat- und/oder Copolymerisat-Kunststoffdispersionen einzusetzen, obwohl dies weniger bevorzugt ist. Falls derartige Abmischungen eingesetzt werden, sollte der Gehalt an Polyurethan, bezogen auf Gesamtfeststoff, mindestens 30 Gew.-% betragen. Auch der Zusatz von an sich bekannten chemisch nicht fixierten, vorzugsweise ionischen Emulgatoren ist möglich, im allgemeinen jedoch nicht erforderlich und auch nicht bevorzugt. Schließlich können den beim erfindungsgemäßen Verfahren einzusetzenden Polyurethandispersionen gewünschtenfalls beliebige inerte Zusatzmittel wie Füllstoffe, Weichmacher oder Pigmente einverleibt werden, falls dies in besonderen Fällen angezeigt erscheint.

Bei den beim erfindungsgemäßen Verfahren einzusetzenden biologischen Materialien handelt es sich beispielsweise um Mikroorganismen, Enzyme, Viren und Antikörper der unterschiedlichsten Art oder um Proteine, Peptide oder auch diagnostische Reagentien, sofern diese auf biologischen Prinzipien beruhen.

Interessante Mikroorganismen, die erfindungsgemäß immobilisiert werden können, sind beispielsweise Aspergillus niger, Gluconobacter suboxydans, Gluconobacter oxydans, Escherichia coli, Saccharomyces cerevisiae, Protaminobacter rubrum, Serratia plymuthica, Pseudomonas putida, Cunninghamella elegans, Clostridien wie z.B. Clostridium thermoaceticum, Clostridium kluyveri, Clostridium butyricum, Clostridium sporogenes, Bacillus licheniformis, Streptomyces olivaceus.

Bei der Durchführung des erfindungsgemäßen Verfahrens wird das biologische Material in in Wasser gelöster oder dispergierter (suspendierter) Form eingesetzt, wobei der Feststoffgehalt der Lösungen oder Dispersionen im allgemeinen bei 3 bis 20 Gew.-% liegt.

Das erfindungsgemäße Verfahren besteht darin, die Lösung bzw. Dispersion des biologischen Materials mit der Polyurethandispersion oder auch -lösung zu vermischen, wobei man das Gewichtsverhältnis, jeweils bezogen auf Trockensubstanz von Polyurethan zu biologischem Material innerhalb eines weiten Bereichs variieren kann. Im allgemeinen liegt dieses Verhältnis bei 20:1 bis 1:5, vorzugsweise bei 10:1 bis 2:1. Nach der Herstellung dieses Gemischs, wird das dispergiert bzw. gelöst vorliegende Polyurethan koaguliert. Diese Koagulation kann nach beliebigen, an sich bekannten Methoden herbeigeführt werden. So kann die Koagulation thermisch induziert werden, sie wird jedoch vorzugsweise unter Verwendung von ionischen Koagulantien bewirkt. Bei Verwendung von kationischen Polyurethandispersionen kann beispielsweise $K_4$-[Fe(CN)$_6$] als wäßrige, beispielsweise 5 gew.-%ige Lösung verwendet werden. Bei Verwendung von anionischen Polyurethandispersionen können beispielsweise entsprechende wäßrige Lösungen von mehrwertigen Metallen und starken Säuren, insbesondere der wasserlöslichen Chloride, Bromide, Nitrate oder Sulfate von Calcium, Magnesium, Barium, Zink oder Eisen zur Anwendung kommen. Zur Koagulation elektrolytempfindlicher Dispersionen sind auch Salze des Natriums, Kaliums oder Ammoniums geeignet.

Besonders bevorzugt ist eine Ausführungsform der Koagulation, derzufolge man die Koagulation durch Einwirkung einer entgegengesetzt geladenen Polyurethandispersion bewirkt. So ist es beispielsweise möglich, zur Koagulation einer Dispersion eines anionisch modifizierten Polyurethans eine Dispersion eines kationisch modifizierten Polyurethans oder auch umgekehrt zu verwenden. Hierbei werden die Mengenverhältnisse der jeweiligen Dispersionen so bemessen, daß die Äquivalente an anionischen Gruppen der zu dispergierenden anionischen Dispersion sich zu den Äquivalenten an kationischen Gruppen der als Koagulanz verwendeten kationischen Dispersion zueinander im Verhältnis von 0,9:1 bis 1,1:1, vorzugsweise 1:1 stehen. Das gleiche Verhältnis wird dann gewählt, wenn eine kationische Dispersion mittels einer anionischen Dispersion koaguliert werden soll. Grundsätzlich ist es auch möglich, bei dieser Ausführungsform des erfindungsgemäßen Verfahrens das zu immobilisierende biologische Material in Form einer wäßrigen Lösung oder Suspension auf beide Dispersionstypen aufzuteilen.

Die Koagulation erfolgt im allgemeinen innerhalb des Temperaturbereichs von 1 bis 60°C, vorzugsweise von 3 bis 40°C und ganz besonders bevorzugt von 5 bis 25°C. Insbesondere im Falle der gegenseitigen Koagulation zweier entgegengesetzt geladener Dispersionen kann die Koagulation unter sehr schonenden Bedingungen, beispielsweise innerhalb des Temperaturbereichs von 5 bis 10°C durchgeführt werden.

Bei Verwendung von wäßrigen Elektrolytlösungen der oben beispielhaft genannten Art als Koagulantien erfolgt die Koagulation beispielsweise durch Eintropfen des Gemischs aus Polyurethandispersion oder auch -lösung und Lösung bzw. Suspension des biologischen Materials in die wäßrige Lösung des Koagulans, wobei man sich beispielsweise einer Spritze mit einem Kanülendurchmesser von ca. 0,1 bis 1, vorzugsweise 0,3 bis 0,8 mm bedienen kann.

Die thermische Koagulation kann beispielsweise dergestalt erfolgen, daß man das Gemisch aus Polyurethandispersion oder auch -lösung und wäßriger Lösung oder Suspension des biologischen Materials auf 60 bis 90°C erhitzt.

Die Koagulation unter Verwendung einer entgegengesetzt geladenen Polyurethandispersion kann beispielsweise dergestalt erfolgen, daß man die als Koagulans verwendete Dispersion unter gutem Durchmischen in das zu koagulierende Gemisch portionsweise oder durch Eintropfen einträgt, wobei ein feinteiliges Koagulat entsteht, welches sich am Boden des Mischgefäßes absetzt.

Grundsätzlich ist im Rahmen der Erfindung unter "Koagulation" die Überführung des im Wasser dispergierten oder auch gelösten Polyurethans in einen, in Wasser nicht mehr dispergierbaren oder löslichen Zustand zu verstehen. Diese Überführung in den nicht mehr dispergierbaren oder löslichen Zustand (Koagulation) kann auch dergestalt erfolgen, daß man das zu koagulierende Gemisch durch Trocknen, beispielsweise bei 20 bis 60°C vom Wasser befreit, so daß das ursprünglich dispergiert oder auch gelöst vorgelegene Polyurethan in einer nicht mehr redispergierbaren oder löslichen Form anfällt. Die bei dieser Trocknung anfallende Trockensubstanz stellt ebenfalls ein erfindungsgemäß immobilisiertes biologisches Material dar und kann, falls die Trocknung beispielsweise durch Filmbildung auf einer Oberfläche durchgeführt wurde, gewünschtenfalls mechanisch zerkleinert werden. Eine ebenfalls bevorzugte Ausführungsform der Erfindung besteht darin, das wäßrige Gemisch auf ein teilchenförmiges oder faserförmiges Substrat aufzubringen und zu trocknen.

Besonders bevorzugt wird die Koagulation so durchgeführt, daß das Koagulat in Form von Teilchen mit einem mittleren Teilchendurchmesser von 0,1 bis 10, vorzugsweise von 0,3 bis 5 mm anfällt. Die Teilchengröße kann beispielsweise durch die Art des Zusammenbringens des zu koagulierenden Gemischs aus Polyurethandispersion und wäßriger Lösung bzw. Suspension des biologischen Materials mit dem Koagulans eingestellt werden. Grundsätzlich ist es jedoch auch möglich, das erfindungsgemäße Verfahren, d.h. die Koagulation so zu führen, daß das immobilisierte biologische Material in Form von Folien, Filmen oder Formteilen oder auch als imprägniertes Fasermaterial aus Glas-, Stein-oder Textilfasern anfällt.

Wie bereits oben ausgeführt kann das Mengenverhältnis von Polyurethandispersion zu biologischem Material innerhalb weiter Bereiche variiert werden. Dies bedeutet in der Praxis, daß sowohl große Mengen an wäßrigen Lösungen oder Dispersionen des biologischen Materials in einer relativ kleinen Menge Polyurethan eingeschlossen werden können, als auch daß andererseits beispielsweise Fermenterbrühen direkt oder nach Konzentrierung z.B. durch Mikrofiltration oder Zentrifugieren erfindungsgemäß immobilisiert werden können.

Die erfindungsgemäßen immobilisierten biologischen Materialien, d.h. die erfindungsgemäßen Verfahrensprodukte können nach ihrer Herstellung, die, wie oben ausgeführt, vorzugsweise durch Koagulation in wäßrigem Milieu erfolgt, durch Dekantieren, Filtrieren, Zentifugieren oder eine andere bekannte Trennungsoperation von der Mutterlauge abgetrennt werden und können, oftmals in noch feuchtem Zustand, gegebenenfalls nach Waschen mit Wasser oder einer Salzlösung der erfindungsgemäßen Verwendung zugeführt werden. Es handelt sich bei den erfindungsgemäßen Verfahrensprodukten um koagulierte Polyurethanionomere, in denen das biologische Material in feinverteiltem bzw. dispergiertem und immobilisiertem Zustand vorliegt.

Die erfindungsgemäßen immobilisierten biologischen Materialien können in wäßrigem Milieu oder auch in organischem Milieu der erfindungsgemäßen Verwendung zugeführt werden.

Vorzugsweise handelt es sich bei den "biologischen Materialien" um bekannte Biokatalysatoren, die in erfindungsgemäß immobilisierter Form als Katalysatoren für Biotransformationen verwendet werden können. Für derartige Umsetzungen sind verschiedene Reaktoren geeignet.

Geeignete Reaktoren sind beispielsweise der Rührtank-Reaktor, der als "batch"-System oder im kontinuierlichen Durchlauf betrieben werden kann. Der immobilisierte Biokatalysator befindet sich im Rührkessel und wird nach beendeter Reaktion entfernt. Bei kontinuierlicher Arbeitsweise wird der immobilisierte Biokatalysator im Reaktor zurückgehalten, während das Substrat kontinuierlich zugeführt und das Produkt kontinuierlich entfernt wird. Ein weiterer, für derartige Umsetzungen geeigneter Reaktor ist der Fließbett-Reaktor, bei dessen Verwendung das Substrat von unten nach oben durch das, den immobilisierten Biokatalysator enthaltende Bette so fließt, daß die Partikel des Biokatalysators aufgetrieben werden und eine fluide Phase bilden. Hierbei kann das Substrat mehrmals durch den Reaktor geleitet werden, um den gewünschten Umsatz zu erreichen. Die genannten Umsetzungen können jedoch auch in Röhrenreaktoren durchgeführt, die mit dem immobilisierten biologischen Material befüllt sind und von dem flüssigen Substrat durchströmt werden.

In den nachfolgenden Beispielen beziehen sich alle Prozentangaben auf Gewichtsprozente.

In den nachfolgenden Beispielen werden folgende Polyurethandispersionen eingesetzt:

Polyurethandispersion 1 (anionisch)

2000 g (1,00 Mol) endständige Hydroxylgruppen aufweisendes Polytetrahydrofuran werden bei 110°C im Wasserstrahlvakuum entwässert. Nach Abkühlen auf 60 -70°C versetzt man mit 289,3 g (1,722 Mol) 1,6-Diisocyanatohexan und erwärmt auf 95 - 100°C. Bei dieser Temperatur wird 2 h nachgerührt. Das erhaltene Produkt wird bei 180°C und 0,04 mbar gedünnschichtet. Man erhält ca. 2150 g eines Prepolymers vom NCO-Gehalt 2,12 %.

300 g obigen Prepolymers (= 0,075 Mol NCO) werden in 610 g Aceton 33 %ig gelöst. Bei 50°C wird nun mit 12,21 g (0,0325 Mol) 50,6 %iger wäßriger Lösung des Na-Salzes der 2-Aminoethyl-2-aminoethansulfonsäure (Na-AAS) versetzt. Nach 15 Min. wird mit 710 ml entsalztem Wasser dispergiert und anschließend das Aceton im Wasserstrahlvakuum destilliert. Man erhält eine stabile, feinteilige Dispersion.

Daten: % $SO_3^-$: 0,85
% Feststoff: 30
pH: 7,5

Polyurethandispersion 2 (anionisch)

300 g des oben beschriebenen Prepolymers werden in 610 g Aceton gelöst und bei 50°C mit 9,34 g (0,0249 Mol) 50,6 %iger wäßriger Na-AAS-Lösung versetzt. Nach 15 Min. dispergiert man mit 708 ml entsalztem Wasser. Das Aceton wird im Wasserstrahlvakuum abdestilliert. Man erhält eine stabile Dispersion.

Daten: % $SO_3^-$: 0,65
% Feststoff: 30
pH: 7,3

Polyurethandispersion 3 (anionisch)

Aus 2000 g (1,00 Mol) Polytetrahydrofuran und 305,8 g (1,82 Mol) 1,6-Diisocyanatohexan wird wie oben beschrieben ein Prepolymer hergestellt. Das erhaltene Prepolymer wird nicht gedünnschichtet und hat einen NCO-Gehalt von 2,82 %.

Nach Lösen in 4680 g Aceton wird bei 50°C mit 227,92 g (0,607 Mol) 50,6 %iger wäßriger Na-AAS-Lösung versetzt und 13 Min. nachgerührt. Nun wird mit 5550 g entsalztem Wasser dispergiert und anschließend das Aceton im Wasserstrahlvakuum destilliert. Man erhält eine sehr feinteilige Dispersion.

Daten: % $SO_3^-$: 2,00
% Feststoff: 30
pH 7,4

Polyurethandispersion 4 (anionisch)

Aus 2000 g (1,00 Mol) Polytetrahydrofuran und 319,2 g (1,90 Mol) 1,6-Diisocyanatohexan wird wie oben beschrieben ein Prepolymer hergestellt. Das Prepolymer hat einen NCO-Gehalt von 3,08 %. Nach Lösen in Aceton (33 %ige Lösung) wird bei 50°C mit 235,4 g (0,627 Mol) 50,6 %iger Na-AAS-Lösung versetzt und nach 15 Min. mit 5560 g entsalztem Wasser dispergiert. Nach Abdestillieren des Acetons erhält man eine sehr feinteilige Dispersion.

Daten: % $SO_3^-$: 2,06
% Feststoff: 30
pH: 7,3

Polyurethandispersion 5 (anionisch)

315 g (0,375 Mol) eines Polyesters auf Basis Adipinsäure/Hexandiol vom Molgewicht 840,9 g (0,1 Mol) 1,4-Butylenglykol werden azeotrop im Wasserstrahlvakuum entwässert. Bei 80°C setzt man 105 g (0,625 Mol) 1,6-Diisocyanatohexan zu und rührt 2 h bei 105 - 110°C nach.

Die Schmelze wird heiß in 1200 ml Aceton gelöst. Bei 50°C wird mit einer Lösung aus 22,5 g (0,0533 Mol) Na-AAS (45 %ig in Wasser) und 60 g entsalztem Wasser versetzt und 5 Min. nachgerührt. Man dispergiert mit 600 ml entsalztem Wasser und destilliert das Aceton im Wasserstrahlvakuum ab. Man erhält 1 kg einer lagerstabilen, feinteiligen Dispersion.

Daten: % $SO_3^-$: 0,97

% Feststoff: 40

pH: 7,5

Polyurethandispersion 6(anionisch)

407,4 g (0,2396 Mol) Hexandiol/Neopentylglykolpolyadipat (Gewichtsverhältnis der Diole = 3:2) werden bei 120°C im Wasserstrahlvakuum entwässert. Bei 70 - 80°C wird mit 77,7 g (0,4625 Mol) 1,6-Diisocyanatohexan versetzt und bei 100°C 1,5 h nachgerührt. Das Prepolymer hat einen NCO-Gehalt von 3,4 %. Nach 33 %igem Einlösen in Aceton wird bei 50°C mit 75,0 g (0,1924 Mol) 2-Aminoethyl-3-aminopropionsäure-Na-Salz (39,5 %ig in Wasser) versetzt und nach 7 Min. mit 1160 ml vollentsalztem Wasser dispergiert. Nach Abdestillieren des Acetons im Wasserstrahlvakuum erhält man eine sehr feinteilige Dispersion.

Daten:    % $COO^-$: 1,6

             % Feststoff: 30

             pH: 7,6

Polyurethandispersion 7 (kationisch)

3725 g (2,192 Mol) eines Polyesterdiols des Molekulargewichts 1700 aus Adipinsäure und einem Gemisch aus 1,6-Dihydroxyhexan und Neopentylglykol im Gewichtsberhältnis 2:3 und 127 g (0,059 Mol) eines Polyethers des Molekulargewichts 2150, hergestellt durch Alkoxylierung von n-Butanol unter Verwendung eines Gemischs aus Ethylenoxid und Propylenoxid im Gewichtsverhältnis EO:PO = 80:20 werden während 1/2 h bei 120°C im Wasserstrahlvakuum entwässert. Bei 60 - 70°C werden dann 551,3 g (2,483 Mol) 5-Isocyanato-1-isocyanatomethyl-1,3,3-trimethylcyclohexan und 417,2 g (2,483 Mol) 1,6-Diisocyanatohexan als Gemisch zugegeben, dann wird auf 100°C erwärmt und solange nachgerührt, bis der berechnete NCO-Wert erreicht ist (ca. 2,5 h). Dann wird in 9100 ml Aceton 40 %ig gelösen. Bei 40-50°C werden 76,3 g (1,526 Mol) Hydrazinhydrat zulaufen lassen, während 10 Min. nachgerührt, 73,7 g (0,508 Mol) Methyl-bis-(3-aminopropyl)-amin in 500 ml Aceton zugetropft, 10 Min. nachgerührt, mit 57,6 g (0,457 Mol) Dimethylsulfat versetzen, 15 Min. nachgerührt und mit 7500 ml entsalztem Wasser innerhalb von 3 Min. dispergiert und dann Aceton im Wasserstrahlvakuum abdestilliert. Man erhält eine feinteilige, stabile kationische PUR-Dispersion.

Daten:    % $N^+$: 0,13

             % Feststoff: 40

             pH: 6

Polyurethandispersion 8 (kationisch)

3000 g (1,765 Mol) des zuletzt genannten Polyesterdiols und 102 g (0,047 Mol) des zuletzt genannten Polyethers werden während 1/2 h bei 120°C im Wasserstrahlvakuum entwässert. Dann werden bei 60 - 70°C 444 g (2,0 Mol) 5-Isocyanato-1-isocyanatomethyl-1,3,3-trimethylcyclohexan und 336 g (2,0 Mol) 1,6-Diisocyanatohexan als Gemisch zugegeben, auf 100°C erwärmt und solange nachgerührt, bis der berechnete NCO-Wert erreicht ist (ca. 2,5 h). Dann wird in 7360 ml Aceton 40 %ig gelöst, bei 40-50°C 48,4 g (0,969 Mol) Hydrazinhydrat zulaufen gelassen, 10 Min. nachgerührt, 95,4 g (0,658 Mol) Methyl-bis-(3-aminopropyl)-amin in 560 ml Aceton zugetropft, 10 Min. nachgerührt, mit 66,6 g (0,592 Mol) DL-Milchsäure (80 %ig in $H_2O$) in 510 ml Wasser versetzt, 5 Min. nachgerührt und mit 5600 ml entsalztem Wasser innerhalb von 11 Min. dispergiert. Schließlich wird das Aceton im Wasserstrahlvakuum abdestilliert.

Daten:    % $N^+$: 0,204

             % Feststoff: 40

             pH: 6

Vergleichsbeispiel 1

Man gibt 0,2 g einer 50 %igen frischen Hefesuspension in Wasser zu 100 ml 10 %iger wäßriger Glukoselösung (enthaltend 0,9 % Kochsalz). Die Suspension wird bei Raumtemperatur unter langsamen Rühren der alkoholischen Gärung überlassen. Nach 5 Tagen erhält man 13,3 % Ethanol, nach 10 Tagen 23,4 % Ethanol (quantitative Gaschromatographie).

Vergleichsbeispiel 2 (gemäß H.J. Rehen, G. Reed, Biotechnology, Band 3 (1983), 341 ff)

Eine Mischung aus 0,2 g 50 %iger wäßriger Bäckerhefesuspension, 0,25 g Na-Alginat und 2 ml Wasser wird bei Raumtemperatur aus einer Einwegspritze Kanülendurchmesser 0,55 mm) in eine 5 %iger Calciumchloridlösung eingetropft. Nach ca. 20 Min. wird das kugelförmige Koagulat isoliert, mit destilliertem Wasser gewaschen und in 100 ml 10 %ige Glukoselösung eingebracht. Die alkoholische Gärung ergibt nach 5 Tagen 16,7 % Ethanol, nach 10 Tagen 26,9 % Ethanol.

Der Vergleichsversuch 2 zeigt anhand der Umsätze, daß die immobilisierte Hefe eine deutlich höhere Aktivität als die frei suspendierte Hefe aufweist (115 % respektive 125,6 % bezogen auf den O-Versuch). Nachteil: Die Alginatkugeln werden nach 2 - 4 Wochen weich und zerfallen.

Beispiele 1 - 6

In 6 Parallelversuchen gibt man jeweils zu 8,33 g der Polyurethandispersionen 1 bis 6 2 g einer 50 %igen wäßrigen Hefesuspension. Jeweils 1/10 der hierbei erhaltenen Gemische werden anschließend durch eine Einwegspritze mit einem Kanülendurchmesser von 0,55 mm langsam in jeweils 250 ml einer 5 %igen Calciumchloridlösung eingetropft. Man erhält jeweils ein kugelförmiges Koagulat mit einem mittleren Teilchendurchmesser von 2 mm. Die Koagulate werden jeweils 3 mal mit je 100 ml entsalztem Wasser auf einem Sieb gut gewaschen und jeweils in 100 ml einer wäßrigen Lösung eingebracht, die neben 10 % Glukose 0,9 % Natriumchlorid enthält.

In den so erhaltenen Gärungseinsätzen liegen somit jeweils 0,25 g Polyurethan (fest), 0,1 g Hefezellen (feuchte Originalmasse) und 100 ml der besagten wäßrigen Lösung vor.

Die Lösungen werden jeweils bei 30°C gelagert und die Menge des nach 5 bzw. 10 Tagen entstandenen Ethanols bestimmt. Die Ergebnisse sind in der nachfolgenden Tabelle zusammengefaßt. Die Prozentangaben bezüglich des Ethanols beziehen sich hierbei auf die Ethanolausbeute, bezogen auf eingesetzte Glukose. Eine vollständige Überführung der Glukose in Ethanol würde einer Ausbeute von 100 % entsprechen. Die Ergebnisse von Vergleichsbeispiel 2 werden ebenfalls nochmals in der Tabelle dargestellt.

## Tabelle

### PUR-Ionomeren

| | $\%\ SO_3^-$ | $\%\ COO^-$ | $\%$ Ethanol bei 30°C 5 d | 10 d |
|---|---|---|---|---|
| Beispiel 1 | 0,85 | - | 15,7 | 25,6 |
| Beispiel 2 | 0,65 | - | 17,5 | 24,9 |
| Beispiel 3 | 2,00 | - | 17,7 | 29,6 |
| Beispiel 4 | 2,07 | - | 16,4 | 28,1 |
| Beispiel 5 | 0,97 | - | 16,0 | 28,9 |
| Beispiel 6 | - | 1,60 | 16,4 | 24,9 |
| Alginat-Vergleich | - | - | 18,7 | 29,6 |

Beispiel 7

Man verfährt völlig analog zu den Beispielen 1 bis 6, jedoch unter Verwendung der Polyurethandispersion 7 und einer 5 %igen wäßrigen Lösung von $K_4[Fe(CN)_6]$ als Koagulanz. Außerdem wird eine salzfreie Glukoselösung verwendet.

Man erhält schließlich einen Gärungsansatz, der aus 0,25 g Polyurethan (fest), 0,1 g Hefe (Feuchtmasse) und 100 ml 10 %ige Glukoselösung besteht. Der Gärungsansatz wird bei 30°C gelagert. Nach 5 Tagen entstanden 14,6 % der theoretisch zu erwartenden Ethanolmenge, nach 10 Tagen 20,6 % und nach 15 Tagen 26,8 %.

Beispiel 8

100 g der Polyurethandispersion 6 wird bei Raumtemperatur mit 24 g 50 %iger Hefesuspension gut durchmischt. Die Mischung wird auf eine Silikonkautschuk-Platte gegossen und bei Raumtemperatur getrocknet.

Der so erhaltene Film mit einer Trockenfilmstärke von 1 mm wurde in Stücke von ca. 1 cm$^2$ geschnitten und mit 1200 ml 10 %iger Glukoselösung vermischt. Nach 14-tägigem Rühren bei Raumtemperatur sind 28,83 % der theoretisch zu erwartenden Menge an Ethanol entstanden.

Beispiel 9

39,5 g der Polyurethandispersion 7 werden mit 15 ml entsalztem Wasser verdünnt und mit 24 g 50 %iger Hefesuspension versetzt. Nach gutem Durchmischen werden 45,4 g der Polyurethandispersion 5 zugetropft. Man erhält ein feinteiliges Koagulat, welches sich nach einiger Zeit am Boden absetzt. Das überstehende, klare Wasser wird abdekantiert und nach zweimaligem dekantierenden Waschen durch 1200 ml 10 %ige Glukoselösung ersetzt. Nach 14 Tagen Gärung bei Raumtemperatur erhält man 29,61 % der theoretisch zu erwartenden Menge an Ethanol.

Beispiel 10

36,1 g der Polyurethandispersion 8 werden mit 25 ml entsalztem Wasser verdünnt und mit 24 g 50 %iger Hefesuspension versetzt. Nach gutem Durchmischen werden 38,9 g Polyurethandispersion 5 zugetropft. Jetzt arbeitet man völlig analog Beispiel 9. 14 Tage Gärung bei Raumtemperatur ergaben 29,16 % Ethanol.

Beispiel 11

10 g der Polyurethandispersion 5 werden mit 16 g einer 50 %igen Suspension von Bäckerhefe versetzt und innig gemischt. Nun setzt man portionsweise 10 g der Polyurethandispersion 8 zu und erhält ein feinteiliges Koagulat, welches auf einem 80 $\mu$-Filter mit destilliertem Wasser gewaschen wird.

15 g des so erhaltenen Biokatalysators werden in einem 200 ml Erlenmeyerkolben mit 50 ml 0,01 M Kaliumphosphat, pH 8,0 und 1 mM hydriertes Nikotinsäureamid (NADH, Grad II der Firma Boehringer, Mannheim) bei 25°C auf einer Rundschüttelmaschine inkubiert.

Die NADH-Oxidase Aktivität der immobilisierten Hefezellen wird über die Abnahme des NADH-Gehalts photometrisch gegen eine Blindprobe ohne Hefezellen bestimmt.

15 g immobilisiertes Präparat besitzen eine Aktivität von 0,27 U (1 U = 1 $\mu$Mol NADH/Min. oxidiert). Diese Reaktion ist zur NAD-Regenerierung in gekoppelten Enzymsystemen anwendbar.

**Patentansprüche**

1. Verfahren zur Immobilisierung von biologischem Material in einer Polyurethan-Matrix, dadurch gekennzeichnet, daß man eine wäßrige Lösung oder Dispersion des biologischen Materials mit einer wäßrigen Polyurethanionomer-Dispersion oder -Lösung mischt und anschließend die Mischung koaguliert.

2. Verfahren gemäß Anspruch 1, dadurch gekennzeichnet, daß man als Polyurethanionomer ein Anionomer oder ein Kationomer enthaltend 15 bis 300 Milliäquivalente pro 100 g an Carboxylat- oder Sulfonatgruppen bzw. an quatären Ammoniumgruppen verwendet.

3. Verfahren gemäß Anspruch 1 und 2, dadurch gekennzeichnet, daß man als biologisches Material Mikroorganismen, Enzyme, Viren, Antikörper, Proteine, Peptide oder diagnostische Reagentien, die auf biologischen Reaktionen basieren, verwendet.

4. Verfahren gemäß Anspruch 1 bis 3, dadurch gekennzeichnet, daß man als biologisches Material Biotransformationen beschleunigende Mikroorganismen verwendet.

**5.** Teilchenförmige gemäß Anspruch 1 bis 4 erhaltene Koagulate, bestehend aus einem koagulierten Polyurethanionomer und darin dispergiertem und immobilisiertem biologischem Material.

**6.** Teilchenförmige Koagulste gemäß Anspruch 5, dadurch gekennzeichnet, daß das biologische Material aus einem Biotransformationen beschleunigenden Mikroorganismus besteht.

**7.** Verwendung der teilchenförmigen Koagulate gemäß Anspruch 6 als Katalysator bei der Durchführung von Biotransformationen.

## Claims

**1.** A process for immobilizing biological material in a polyurethane matrix, characterized in that an aqueous solution or dispersion of the biological material is mixed with an aqueous polyurethane ionomer dispersion or solution and the mixture is subsequently coagulated.

**2.** A process as claimed in claim 1, characterized in that an anionomer or a cationomer containing 15 to 300 milli-equivalents per 100 g carboxylate or sulfonate groups or quaternary ammonium groups is used as the polyurethane ionomer.

**3.** A process as claimed in claims 1 and 2, characterized in that microorganisms, enzymes, viruses, antibodies, proteins, peptides or diagnostic reagents based on biological reactions are used as the biological material.

**4.** A process as claimed in claims 1 to 3, characterized in that biotransformation-accelerating microorganisms are used as the biological material.

**5.** Particulate coagulates obtainable by the process claimed in claim 1 consisting of a coagulated polyurethane ionomer and biological material dispersed and immobilized therein.

**6.** Particulate coagulates as claimed in claim 5, characterized in that the biological material consists of a biotransformation-accelerating microorganism.

**7.** The use of the particulate coagulates claimed in claim 6 as a catalyst for carrying out biotransformations.

## Revendications

**1.** Procédé d'immobilisation d'une matière biologique dans une matrice de polyuréthane, caractérisé en ce qu'on mélange une solution ou une dispersion aqueuse de la matière biologique avec une dispersion ou une solution aqueuse d'un ionomère de polyuréthane puis qu'on coagule le mélange.

**2.** Procédé selon la revendication 1, caractérisé en ce qu'on utilise comme ionomère de polyuréthane un ionomère anionique ou un ionomère cationique contenant respectivement, pour 100 g, de 15 à 300 milliéquivalents de groupes carboxylate ou sulfonate ou de groupes ammonium quaternaire.

**3.** Procédé selon les revendications 1 et 2, caractérisé en ce qu'on utilise comme matière biologique des microorganismes, des enzymes, des virus, des anticorps, des protéines, des peptides ou des réactifs de diagnostic se basant sur des réactions biologiques.

**4.** Procédé selon les revendications 1 à 3, caractérisé en ce qu'on utilise comme matière biologique des microorganismes qui accélèrent des biotransformations.

**5.** Coagulats particulaires obtenus selon les revendications 1 à 4, constitués par un ionomère de polyuréthane coagulé et une matière biologique qui y est dispersée et immobilisée.

**6.** Coagulats particulaires selon la revendication 5, caractérisés en ce que la matière biologique est constituée par un microorganisme qui accélère des biotransformations.

7. Utilisation des coagulats particulaires selon la revendication 6 comme catalyseurs pour la réalisation de biotransformations.